# EUROPEAN PATENT SPECIFICATION

(11) **EP 1 811 865 B1**
(45) Date of publication and mention of the grant of the patent: **16.07.2008**
(21) Application number: 04808740.7
(22) Date of filing: 26.11.2004
(51) Int. Cl.: A23L 1/29

(54) **INFANT NUTRITION WITH PROTEASE INHIBITOR**
KLEINKINDERNAHRUNG MIT PROTEASEHEMMER
ALIMENT POUR NOURRISSON CONTENANT UN INHIBITEUR DE PROTEASE

(43) Date of publication of application: 01.08.2007
(73) Proprietor: N.V. Nutricia, 2712 HM Zoetermeer (NL)
(72) Inventor: VAN LAERE, Katrien, Maria, Jozefa, NL-6666 WS Heteren (NL); DELSING, Bernardina, Johanna, Martina, NL-5246 XH Rosmalen (NL); BEERMANN, Christopher, 61267 Neu-Anspach (DE); RAGGERS, Rene, John, NL-1057 NX Amsterdam (NL)
(74) Representative: van Westenbrugge, Andries
(86) International application number: PCT/NL2004/000824
(87) International publication number: WO 2006/057551

(56) References cited:
- WO-A-93/10818
- WO-A-03/092603
- US-A1- 2002 119 915
- US-A1- 2004 076 719

## Description

### FIELD OF THE INVENTION

The present invention relates to nutrition, especially nutritionally complete infant nutrition, and the use of such nutrition for preventing and/or treating childhood obesity.

### BACKGROUND OF THE INVENTION

Childhood obesity is an increasing problem in developed countries. For instance, in the United States in the year 2000 about 15% of the children up to age 11 were considered to be obese, whereas in 1980 this was 7%. Also in Europe an increase in childhood obesity is observed.

Obese children are very likely to have obesity persist into adulthood. Childhood obesity is associated with elevated blood pressure and lipids, and increased risk of diseases, such as asthma, type 2 diabetes, arthritis, and cardiovascular diseases at a later stage of life. Furthermore, childhood obesity can have a negative psycho-social effect. Causes of childhood obesity include lack of regular physical exercise, sedentary behaviour, eating habits, socio-economic factors and genetic factors. Also early nutrition plays an important role in the prevention of childhood obesity. Armstrong & Reilly (2002, Lancet 359:2003-4) observed that feeding human milk lowers the risk of childhood obesity.

Breast-feeding is the preferred method of feeding infants. However, there are circumstances that make breast-feeding impossible or less desirable. In those cases infant formula and follow-on formula are a good alternative. The composition of modem infant formula and follow-on formulas is adapted in such a way that it meets the special nutritional requirements of the growing and developing infant. A formula fed infant is (almost) completely dependent on the formula for its nutrients and water. Therefore, the normal remedies for obesity, e.g. increase of satiety or decrease of appetite, or an increase in thermogenesis, are not feasible, since the strict nutritional needs of the infant are imperilled.

WO 0022937 describes the use of a protein material whereof the digestion speed has been reduced for preparing a composition for enteral administration enabling to modulate the post-prandial plasma amino acid level. This document also describes a composition for enteral administration to a mammal containing a protein material whereof the digestion speed has been reduced. Documents US 2002/119915, US 2004/076719 and WO93/10818 disclose compositions comprising a protease inhibitor together with proteins, oils/fats and carbohydrates.

There is a need or an infant nutrition that meets all nutritional requirements to support an optimal growth and development, and prevents the occurrence of childhood obesity later in life.

### SUMMARY OF THE INVENTION

Feeding infants with infant formula results in a higher post-prandial insulin response than when the infants receive human milk, while blood glucose levels are not lowered when infant formula is administered. Therefore, increased post-prandial insulin levels as a result of feeding infant formula, compared to feeding human milk, are undesirable, since they induce a form of insulin resistance in formula fed infants, which contributes to the development of childhood obesity.

The inventors surprisingly found that the administration of an infant nutrition comprising a protease inhibitor resulted in lower post-prandial levels of insulin and lower post-prandial glucose levels compared to the administration of the same infant nutrition that does not comprise the protease inhibitor, while at the same time a sufficient level of plasma amino acids, in particular essential amino acids, was achieved. The post-prandial insulin response, the post-prandial blood glucose levels and the post-prandial levels of plasma amino acids observed after administration of the infant nutrition with protease inhibitor were comparable to those observed when feeding human milk.

Hence, the present infant nutrition with protease inhibitor, can be advantageously used in a method for the treatment and/or prevention of childhood obesity. The present invention can also be suitably used in a method for the treatment and/or prevention of secondary disorders in children suffering from childhood obesity, particularly one or more of the secondary disorders selected from the group consisting of diabetes, cardiovascular diseases, hypertension, asthma, sleep apnoea, orthopaedic complications (especially of the leg and hip bones), and arthritis.

### DETAILED DESCRIPTION OF THE INVENTION

One aspect of the invention relates to a composition, which comprises proteins, carbohydrates and at least one source of fat selected from the group consisting of vegetable fat, marine fat and microbial fat, said composition comprising one or more protease inhibitors, selected from the group consisting of trypsin inhibitors, chymotrypsin inhibitors, and elastase inhibitors. Preferably the protease inhibitor is selected from the group consisting of trypsin and chymotrypsin inhibitors. In one embodiment the composition comprises at least vegetable fat.

A second aspect of the invention is the use of the composition according to the present invention for the manufacture of a nutritional composition for providing nutrition to an infant.

A third aspect of the invention is the use of the composition according to the present invention for the manufacture of a preparation for use in a method to prevent and/or treat childhood obesity, said method comprising orally administering the preparation to an infant.

A further aspect of the invention is the use of present a composition for the manufacture of a nutritional composition for use in a method for the treatment and/or prevention of diabetes, cardiovascular diseases, hypertension, asthma, sleep apnoea, orthopaedic disorders and/or arthritis in a subject suffering from childhood obesity, said method comprising orally administering the nutritional composition to an infant.

Another aspect of the invention is a process for preparing an infant nutrition, comprising admixing:
a. a source comprising at least one protease inhibitor, selected from the group consisting of trypsin inhibitor, chymotrypsin inhibitor, and elastase inhibitor;
b. a fat source;
c. a protein source; and
d. a carbohydrate source.

Yet another aspect of the invention is the use of at least one protease inhibitor, selected from the group consisting of trypsin inhibitor, chymotrypsin inhibitor, and elastase inhibitor, for the manufacture of a nutritional composition for the prevention and/or treatment of childhood obesity.

The term infant as used herein refers to a human with an age from 0 to 24 months. A child is a human with an age from 0 to 12 years. In a preferred embodiment, the present invention relates to the prevention of childhood obesity in infants with an increased risk of developing childhood obesity.

The term "childhood obesity", as used in the present invention, refers to obesity or overweight of children. Both infants and children can suffer from childhood obesity. Particularly, children with a gender specific BMI-for age above the 85^{th} percentile, or even above the 95^{th} percentile are suffering from childhood obesity. BMI (body mass index) is an anthropometric measure, defined as weight in kilograms divided by the square of length in metres. Tables with gender specific BMI-for age are publicly available for instance at the US National Center for Health Statistics. If a child has a gender specific BMI-for age above the 85^{th} percentile or even 95^{th} percentile, this means that 85 % or even 95 % of the population consisting of children with the same age and sex have a lower BMI.

Wherever in this description a composition is described in terms of a volume, this is meant to refer to a ready-to-drink liquid composition which does not require further dilution to make it suitable to be administered to a child, in particular an infant.

### Protease inhibitors

The term "protease" as used herein refers to enzymes which are capable of hydrolysing proteins and/or peptides by cleavage of the peptide bond. The present invention particularly relates to the inhibition of human intestinal proteases trypsin, chymotrypsin and/or elastase.

The term "protease inhibitor" as used in connection with the present invention refers to a compound, substance and/or composition which is capable of inhibiting the action of protease. The present protease inhibitor is preferably food grade and non-toxic. The present invention particularly relates to protease inhibitors capable of inhibiting the activity of human intestinal trypsin, human intestinal chymotrypsin and/or human intestinal elastase. The compound, substance and/or composition capable of inhibiting the action of trypsin, chymotrypsin and/or elastase can be easily identified by the skilled person using methods known in the art, e.g. using the methods that are described hereinbelow.

Preferably, the present composition comprises at least one of the following ingredients as protease inhibitor:
- ovomucoid (derived from avian eggs);
- antitrypsin and/or antichymotrypsin as found in mammalian blood, plasma, milk and/or colostrum as well as in organs from non-human mammals;
- recombinant human α₁-antitrypsin, recombinant human inter-α-trypsin inhibitor, and/or recombinant human α₁-antichymotrypsin;
- protease inhibitor from fungi and/or bacteria, preferably actinomyces, streptomyces, *Bifidobacteria*, or lactic acid bacteria;
- protease inhibitor as found in corn kernels, potatoes and/or from leguminous seeds such as lima beans, chick peas, garden beans, adzuki beans and/or soy beans;
- zinc gluconate;
- or mixtures of any one of the above mentioned preparations.
Preferably, the protease inhibitor is obtained from a source selected from the group consisting of potato, leguminous seeds, non-human milk, avian egg white and microbial culture. Preferably, the protease inhibitor is a peptide. Protease inhibitors can be obtained commercially for example from Sigma-Aldrich or can isolated, for example described by Seidl & Liener, (1972) J. Biol. Chem. 247:3533-3538, and Michael et al, 1976, Proc. Natl. Acad Sci, 73:1941-1944 and in WO02060932. In a particular embodiment the protease inhibitor is zinc gluconate.

It is preferred that the composition comprises at least one protease inhibitor selected from the group consisting of α₁-antitrypsin as found in human plasma; α₁-antichymotrypsin as found in human plasma; aprotinin as found in bovine lung; Kunitz inhibitor as found in bovine pancreas or bovine colostrum; Kazal inhibitor, as found in bovine pancreas; Bowman-Birk trypsin-chymotrypsin inhibitor, as found in soy bean; trypsin inhibitor type I-S, as found in soy beans; trypsin inhibitor type II-S as found in soy beans; Kunitz inhibitor, as found in soy bean; trypsin inhibitor as found in lima beans; trypsin inhibitor type II-L, as found in lima beans; Hageman factor, as found in corn kernels; ovoinhibitor, as found in chicken egg white; trypsin inhibitor type III-O, as found in chicken egg white; trypsin inhibitor type IV-O, as found in chicken egg white; trypsin inhibitor type II-T, as found in turkey egg white; streptomyces subtilisin inhibitor; serpin as produced by *Bifidobacteria*; potato inhibitor I; potato inhibitor II; and zinc gluconate. It is especially preferred that the composition comprises at least one protease inhibitor selected from the group consisting of α₁-antitrypsin, as found in human plasma; α₁-antichymotrypsin as found in human plasma; Kunitz trypsin inhibitor, as found in bovine colostrum; Bowman-Birk trypsin-chymotrypsin inhibitor, as found in soy bean; ovoinhibitor, as found in chicken egg white; serpin as produced by *Bifidobacteria*; potato inhibitor I; and potato inhibitor II.

### Concentrations and activities of protease inhibitors

Trypsin activity and chymotrypsin activity can be measured according to the method described by Schwert G.W. and Takenaka Y. (Biochim. Biophys. Acta (1955) 16, 570). In the method for determination of trypsin activity N-benzoyl-L-arginine ethyl ester [BAEE] is hydrolysed at the ester linkage causing an increase of absorbance measured at 253 nm and 25^{°}C. In the method for determining chymotrypsin activity, N-Acetyl-L-Tyrosine Ethyl Ester [ATEE] is hydrolysed at the ester linkage causing a decrease of absorbance measured at 237 nm and 25^{°}C. Elastase activity can be measured by hydrolysis of N-acetyl-(L-ala)₃-methyl ester (AAAAME) as described by Gertler, A. and Hofmann, T. (1970). Can. J. Biochem. 48, 384-6.

One unit of trypsin activity is defined as the hydrolysis of 1 µmol BAEE per minute under the reaction conditions as described in Schwert (*supra*). One unit of chymotrypsin activity is defined as the hydrolyses of 1 µmol ATEE per minute under the reaction conditions as described in Schwert (*supra*). One unit of elastase activity as used herein is defined as hydrolysis of 1 µmol of AAAAME per minute under the reaction conditions as described in Gertler (*supra*).

One unit of trypsin inhibitor activity (TIU) is defined as the level of activity that inhibits one unit of trypsin activity. One TIU inhibits 50 % of the activity of a trypsin preparation which has an activity of 2 units. Analogously, one unit of chymotrypsin, and elastase inhibitor are the activities of inhibitors that inhibit one unit of chymotrypsin activity, and one unit of elastase activity, respectively. One unit of protease inhibitor (PIU) is defined as the amount of inhibitor that inhibits one unit of the sum of activities of trypsin, plus chymotrypsin plus elastase.

Preferably, the protease inhibitor or protease inhibitors is present at an activity of at least 0.75, more preferably at least 3.0, most preferably at least 9.0 of protease inhibitor units of the sum of trypsin, chymotrypsin, and elastase inhibited (PIU) per g dry weight of the composition. Preferably, the protease inhibitor is present in concentrations below 300, more preferably below 75 PIU per g dry weight of the composition. When the composition is in the form of a ready-to-drink liquid, per 100 ml the composition preferably comprises at least 10, more preferably at least 40, most preferably at least 120 PIU.

Preferably the present protein inhibitor does not have a particularly high inhibitory activity per weight of inhibitor, i.e. specific inhibitory activity, as this may result in a high local inhibitory activity, which is undesirable. A certain volume and weight of the protease inhibitor is also needed to obtain proper mixing with the food matrix. Hence the present composition preferably comprises at least 35 µg protease inhibitor, even more preferably at least 140 µg protease inhibitor, most preferably at least 560 µg per g dry weight of composition. Per 100 ml the composition in the form of a ready-to-drink liquid preferably comprises at least 0.5 mg, more preferably at least 2.0 mg, most preferably at least 8.0 mg protease inhibitor. The protease inhibitor used preferably has a specific inhibitor activity of less than 2000, even more preferably less than 400 PIU per mg protease inhibitor, most preferably less than 100 PIU per mg.

However, the specific protease inhibitory activity should also not be too low it than may put restrictions to the nutrients which are advantageously included in the composition for providing nutrition. Hence, the present composition preferably comprises less than 0.2 gram protease inhibitor per 100 ml, more preferably less than 0.050 gram per 100 ml. Alternatively, the present composition preferably comprises less than 14 mg protease inhibitor per g dry weight of the composition, more preferably less than 3.5 mg protease inhibitor per g dry weight of the composition. The specific protease inhibitor activity preferably is above 0.2 PIU/mg, or even more preferably above 1.0, most preferably above 4.0 PIU/mg protease inhibitor.

In order to stay as close as possible to the human milk the present composition preferably contains the same protease inhibitory activity as human milk. Preferably the protease inhibiting activity is within 20 % to 5000 %, more preferably between 40 % and 1000 % of that found in human milk. For determination of the protease inhibitor activity that should be present in the composition, the following method is preferably used:
- Determine the protease inhibitory activity of a solution of 15 mg human serum α₁-antitrypsin (Athena Biochemicals, Athens, GA) and 2.5 mg human serum α₁-antichymotrypsin (ICN Biochemicals, Costa Mesa, CA) per 100 ml in a suitable assay for determining trypsin, chymotrypsin and/or elastase inhibitory activity as described herein above.
- The protease inhibitor activity to be added per 100 ml of the present composition (when in ready-to-use liquid form) is between 20 and 5000 % of the activity above obtained, preferably between 40 and 1000% of this activity.
For determination of weight of the protease inhibitor that should be present in the composition, the following method is preferably used:
- Using the same assay as for determination of the protease inhibitory activity to be added to the present composition, a solution of 17.5 mg of the present protease inhibitor is tested for its inhibitory activity in 100 ml. After calculation of the protease inhibitory activity per weight, the weight of protease inhibitor to be added to the present composition per 100 ml (ready-to used liquid formula) is the weight which provides between 20 and 5000 % of the trypsin, chymotrypsin and/or elastase inhibitory activity obtained from 15 mg human serum α₁-antitrypsin and 2.5 mg human serum α₁-antichymotrypsin inhibitor.
Comparably, the inhibitor activity can be expressed per g dry weight of the composition. In a preferred embodiment the protease inhibitor activity per g dry weight of the composition is 20 to 5000 %, more preferably 40 to 1000 % of that of 1.1 mg human serum α₁-antitrypsin and 0.18 mg human serum α₁-antichymotrypsin.

In a preferred embodiment, the protease inhibitor activity of the composition is 6 to 2500 PIU per g protein, more preferably 25 to 600 PIU per g protein. The protease inhibitor is preferably present at 0.03 mg to 120 mg per g protein, more preferably from 0.12 mg to 30 mg per g protein. Alternatively, the protease inhibitor activity present per g protein is preferably 20 to 5000 %, more preferably 40 to 1000 % of the activity of 8.8 mg human serum α₁-antitrypsin and 1.5 mg human serum α₁-antichymotrypsin.

### Nutrients

In order to provide a nutrition for an infant which meets the nutritional requirements to support growth and development, proteins, carbohydrates and fats are esential. It is highly preferred the nutition further comprises (essential) vitamins, minerals and trace elements. Suitable vitamins, minerals and trace elements are well known and it is common general knowledge to the skilled person which and in what amounts these can be included in nutritional products for infants. For example those vitamins, minerals and trace elements that are present in commercially available infant nutrition such as Nutrilon^{®} 1 and 2 are suitable to be included in the compositions according to the present invention as well.

### Proteins

The present composition comprises proteins. It is preferred that at least 50 wt.%, even more preferably at least 90 wt.% of the protein in the present composition is derived from non human mammalian milk, preferably cow's milk. Casein and whey are preferably present in weight ratio ranging from 20/80 to 80/20. This is an optimal range, since on one hand the amino acid composition of bovine casein is more similar to that found in human milk protein, and on the other hand whey protein is easier to digest and found in greater amounts in human milk. Preferably, at least 80 wt.%, preferably at least 95 wt.% the proteins in the present composition are not hydrolysed by proteases to smaller peptides or free amino acids. When the composition is in a liquid form, it preferably comprises 1.0 to 6.0 g protein per 100 ml, preferably 1.0 to 2.5 g of protein per 100 ml. The composition comparably comprises 7 wt.% to 40 wt.%, preferable 8 wt.% to 20 wt.% protein based on dry weight. The protein content is calculated according to Kjeldahl, with N*6.38, with N being the amount of nitrogen measured. Preferably the amount of protein in the present composition is 5 to 16 en.%, most preferably 8.0 to 12.0 en.%. En.% is short for energy percentage and represents the relative amount each constituent contributes to the total caloric value of the preparation.

### Digestible carbohydrates

The present composition preferably comprises a source of digestible carbohydrates selected from the group consisting of lactose, maltodextrin, starch, fructose, sucrose, glucose and maltose. Since it is important that the insulin response and glycaemic index is low, it is preferred that at least 35 wt.%, preferably at least 50 wt.%, most preferably at least 75 wt.% of the digestible carbohydrate of the present composition is lactose. Preferably the amount of sucrose plus glucose is below 6 wit.%, preferably below 2 wt.% of the digestible carbohydrates. The present composition preferably comprises 25 to 75 en.%, preferably 40 to 55 en.% digestible carbohydrates. When in liquid form, the present composition preferably comprises 6 to 19 g digestible carbohydrates per 100 ml, more preferably 6 to 10 g per 100 ml. Based on dry weight, the composition preferably comprises 40 to 75 wt.% digestible carbohydrates. The term "digestible carbohydrate" as used herein refers to a carbohydrate capable of being converted into units that can be absorbed in mouth, esophagus, stomach and/or small intestine of the human alimentary canal.

### Non-digestible, fermentable carbohydrates

Non-digestible carbohydrates are carbohydrates that enter the human colon intact after oral ingestion.

The term "fermentable" as used herein refers to the capability to undergo (anaerobic) breakdown and conversion by micro-organisms in the lower part of the gastro-intestinal tract (e.g. colon) to smaller molecules, in particular short chain fatty acids and lactate. The fermentability may be determined by the method described in Am. J. Clin. Nutr. 53, 1418-1424 (1991).

Non-digestible, fermentable carbohydrates (NDFC) have a blood glucose tempering effect, because they delay gastric emptying and shorten the small intestinal transit time. This effect may be caused via the short-chain fatty acids produced from the oligosaccharides in the colon via the so called ileocolonic brake, which refers to the inhibition of gastric emptying by nutrients reaching the ileo-colonic junction. Short-chain fatty acids may also shorten ileal emptying. Therefore non-digestible, fermentable carbohydrates and protease inhibitor are believed to synergistically prevent and/or treat childhood obesity.

According to a preferred embodiment the composition comprises one or more non-digestible, fermentable carbohydrates. The composition preferably comprises 0.2 - 1.5 g, preferably 0.3 to 1.0 g NDFC, per 100 ml liquid. The composition comprises preferably, based on dry weight, 1 to 10 wt.%, preferably 2 to 6 wt.%. Preferably, the composition comprises at least one non-digestible, fermentable carbohydrates selected from the group consisting of polyfructose, fructo-oligosaccharides, galacto-oligosaccharides, partially hydrolysed galactomannan, acidic oligosaccharides and resistant or indigestible polydextrin. In an especially preferred embodiment the composition comprises a) a mixture of trans-galacto-oligosaccharides with polyfructose; or b) a mixture of partially hydrolysed guar gum with one carbohydrate selected from the group consisting of polyfructose and resistant or indigestible polydextrin, since these mixtures synergistically produce the highest amounts of short chain fatty acids.

Polyfructose is a polysaccharide carbohydrate comprising a chain of β-linked fructose units with a degree of polymerisation of 10 or more. Polyfructose includes inulin, levan and/or a mixed type of polyfructan. Inulin suitable for use in the compositions is also readily commercially available, e.g. Raftiline®HP (Orafti).

Fructo-oligosaccharides (FOS) refer to glucose- and/or fructose-terminated fructose chains, with a DP below 10. Thus, FOS can be described as GFₙ₋₁ chains and/or Fₙ chains, wherein G is a glucosyl unit, F is fructosyl unit and n = 1-9. The majority (at least 90 %, preferably at least 95 %) of the fructose units is linked by β (2,1) fructosyl-fructose linkages. A suitable source of FOS is Raftilose® (Orafti), or Actilight (Beghin-Meiji).

Galacto-oligosaccharides (GOS) refers to oligosaccharides comprising galactose units, with a DP of less than 10. A glucose unit may be present at the reducing end of the chain. GOS comprises α- and β-galacto-oligosaccharides. Preferably at least 66 % of the saccharide units of the GOS are galactose units. Trans-galacto-oligosaccharides (TOS) are galacto-oligosaccharides in which the majority of the galactose units (at least 90 %, preferably at least 95 %) are linked by β-bonds, for example β-(1,4 bonds). Preferably, at least 50 % of the bonds of the GOS as used in the present invention are β-bonds. Such a TOS is for example that found in Vivinal®GOS (Borculo Domo Ingredients, Zwolle, Netherlands).

The term acid oligosaccharide refers to oligosaccharides comprising at least one acidic group selected from the group consisting of N-acetylneuraminic acid, N-glycoloylneuraminic acid, free or esterified carboxylic acid, sulfuric acid group and phosphoric acid group. The acidic oligosaccharide preferably comprises uronic acid units (i.e. uronic acid polymer), more preferably galacturonic acid units. The acid oligosaccharide may be a homogeneous or heterogeneous carbohydrate. Preferably hydrolysates of pectin and/or alginate are used. The DP is preferably below 10.

Partially hydrolysed galactomannan refers to a composition in which galactomannan has been subjected to hydrolyses and has not been hydrolysed to its monomeric units. Galactomannan are polysaccharides comprising at least 90 %, preferably at least 95 %, of β-(1,4)-D-mannopyrasyl units in the linear chain, and galactose branches bound thereto via α-(1,4)-D bonds. According to a particularly preferred embodiment guar gum is used. Methods to prepare partially hydrolysed guar gum (PHGG) are described in EP0557627 and EP1252195. PHGG is commercially available under the tradename Benefiber® from Novartis Nutrition Corporation or under the tradename "Sunfiber AG ®" from Taiyo Kagaku, Japan. Preferably, the hydrolysed guar gum is in an agglomerated form, which has better solubility.

Resistant or indigestible polydextrin, refers to indigestible carbohydrates which have a DP of 3 to 50, preferably of 4 to 20 and in which the monomeric units are at least 80 %, preferably at least 85 % originating from glucose (based on the total of monomeric units present). The average degree of polymerisation is between 10 and 16 monosaccharide units per molecule. In a preferred embodiment, the indigestible polydextrins are randomly branched and comprise α-(1,4), α-(1,6) glucosidic bonds and α/β-(1,2), α/β-(1,3),and β-(1,6) linkages. Indigestible polydextrins are for example available under the tradename "Fibersol 2®" from Matsutami Inductries or Litesse® from Danisco.

### Fat

The composition comprises at least one fat source selected from the group consisting of vegetable fats, marine fats and microbial fats.

Saturated fatty acids are prone to oxidation and ingestion leads to obesity in children. Therefore, the amount of saturated fatty acids is preferably below 58 wt.%, most preferably below 45 wt.% of total fatty acids. The concentration of monounsaturated fatty acids preferably ranges from 17 to 60 wt.% based on weight of total fatty acids. The concentration of polyunsaturated fatty acids in the present composition is preferably between 11 and 36 wt.% based on weight of total fatty acids.

The essential fatty acids linolenic acid (LA; an omega 6 fatty acid) and α-linolenic acid (ALA; an omega 3 fatty acid), should be present in sufficient amounts and in a balanced ratio, since LA and ALA deficiency and imbalance are correlated with conditions such as insulin resistance and obesity. The composition therefore preferably comprises 0.3 to 1.5 g LA per 100 ml, and at least 50 mg ALA per 100 ml. Based on dry weight the present composition preferably comprises 1.8 to 12.0 wt% LA, and at least 0.30 wt.% ALA. The weight ratio LA/ALA is preferably between 5 and 15. Preferably the present composition comprises long chain polyunsaturated fatty acids (LC PUFA), more preferably eicosapentaenoic acid (EPA) and/or docosahexaenoic acid (DHA). Both DHA and EPA improve the insulin sensitivity and are therefore advantageously included in the present composition. A fat composition with the properties as described above is believed to act synergistically with the protease inhibitor on the prevention and/or treatment of childhood obesity.

Microbial fat includes fat derived from algae and funghi.

The composition preferably comprises 2.1 to 6.5 g fat per 100 ml when in liquid form. Based on dry weight the composition preferably comprises 12.5 to 30 wt.% fat. The present composition preferably comprises 35 to 60 en.% fat, more preferably 39 to 50 en.% fat.

### Osmolytes

Osmolytes stabilise protease inhibitors such the trypsin inhibitor. Therefore, the osmolytes and protease inhibitor in the present composition have a synergistic effect on prevention and/or treatment of childhood obesity. Preferably, the present composition comprises one or more osmolytes selected from the group consisting of betaine, sarcosine, myo-inositol, taurine, choline, and creatine.

The composition preferably comprises at least 8 mg osmolyte per 100 ml when the present composition is in liquid form. Preferably the composition comprises not more than 90 mg osmolyte per 100 ml when the present composition is in liquid form. The composition preferably comprises at least 0.5 mg osmolyte per g dry weight of the present composition. The composition preferably comprises not more than 6 mg osmolyte per g dry weight of the present composition.

### Liquid composition

The present composition is preferably administered in liquid form. In order to meet the caloric requirements, the composition preferably comprises 50 to 200 kcal/100 ml, more preferably 60 to 90 kcal/100 ml. The osmolarity of the present composition is typically between 150 and 420 mOsmol/l, preferably 260 to 320 mOsmol/l. The low osmolarity aims to reduce the gastrointestinal stress, e.g. reduce the incidence of diarrhoea, particularly in infants.

Preferably the composition is in a liquid ready-to-drink form, with a viscosity below 35 cps. Suitably, the composition is in a powdered from, which can be reconstituted with water to form a liquid, or in a liquid concentrate form, which should be diluted with water

### Daily dosages

When the composition is a liquid form, the preferred volume administered on a daily basis is in the range of about 80 to 2500 ml, more preferably about 450 to 1000 ml per day, which is a suitable amount for an infant.

### Treatment

The present composition can advantageously be used in a method for the treatment and/or prevention of childhood obesity. The present composition can also advantageously be used to treat and/or prevent type 2 diabetes, hypertension, cardiovascular diseases, arthritis, sleep apnoea, asthma, and/or orthopaedic complications (especially of the leg and hip bones), in infants and/or children suffering from childhood obesity.

The present composition is preferably administered orally. The composition is particularly useful in a method for providing nutrients to an infant and/or stimulating the growth of an infant. As the composition is particularly useful for preventing childhood obesity during later stages of life, the composition is advantageously administered to an infant or child of 0-24 months, preferably to an infant or child of 0-18 months.

Packaged nutritional compositions, which have been provided with labels that explicitly or implicitly direct the consumer towards the use of said supplement or product in accordance with one or more of the above or below purposes, are encompassed by the present invention. Such labels may for example make reference to the method for the treatment of childhood obesity by incorporation of terminology like "lean", "prevention of overweight", "development of a healthy body mass" and the like. The childhood obesity preventing properties of the product may be indicated via indicia such as pictures, drawings and other indicia from which a consumer can conclude that the product aims to treat or prevent childhood obesity.

### LEGENDS TO THE FIGURES:

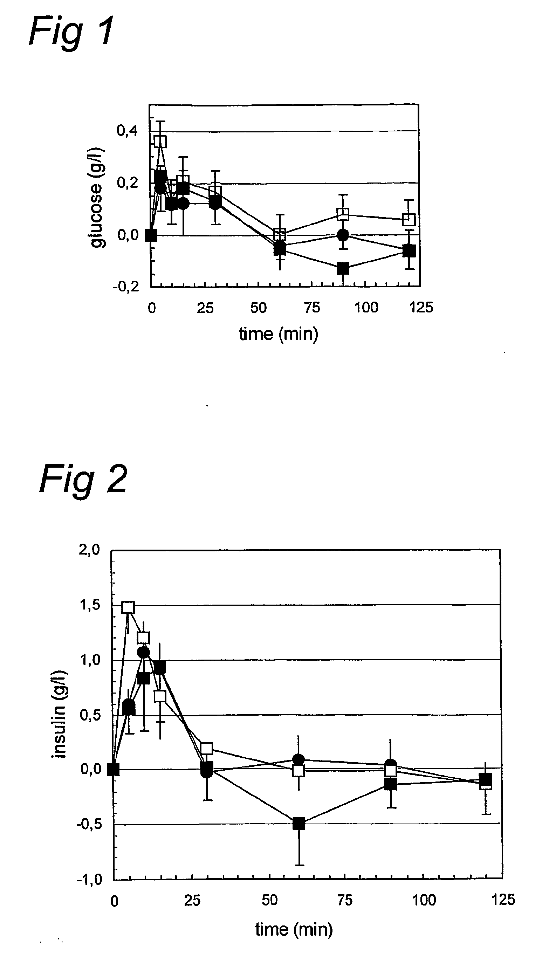

Figure 1A: Post-prandial blood glucose levels in rats fed 2 ml human milk (●), rats fed 2 ml standard infant milk formula (□) and rats fed 2 ml standard infant milk formula with 1 mg soy bean trypsin/chymotrypsin inhibitor (Sigma T9777) (■).
Figure 1B: Post-prandial insulin levels in rats fed 2 ml human milk (●), 2 ml standard infant milk formula (□) and 2 ml standard infant milk formula supplemented with 1 mg soy bean trypsin/chymotrypsin inhibitor (Sigma T9777) (■).

### EXAMPLES

### Example 1

### Animals:

20 adult male Wistar rats (aged 10 weeks at the start of the experiment) were housed individually. The animals had *ad libitum* access to water and food (Standard Rat Chow, Harlan). The animals received a permanent canula in the jugular vein during surgery under isoflurane/N₂O/O₂ anaesthesia, to enable stress-free repeated blood sampling.

### Treatment:

After a 4 h fasting period, 10 animals were fed 2 ml of a milk composition. Three different compositions were tested in a cross-over design (experiments separated by one week).
1 human breast milk
2 Nutrilon^{®} 1
3 Nutrilon^{®} 1 with 1 mg protease inhibitor (Soy bean chymotrypsin and trypsin inhibitorinhibitor, Sigma T9777).
The composition of Nutrilon^{®} 1 is given in table 1. Subsequently, blood samples (200 µl) were collected in heparinised chilled tubes at t=0, 5, 10, 15, 30, 60, 90, and 120 minutes after feeding. Subsequently, plasma was separated after centrifugation (10 min, 5000 rpm) and stored at -20 °C untill analysis.

### Measurement of insulin:

Plasma insulin was measured by radioimmunoassay (RIA, Linco) according to the kit protocol with the following adjustment: all assay volumes were reduced four times.

### Measurement of glucose:

Plasma glucose was measured with an oxidase-peroxidase method in 96-wells format (Roche Diagnostics, #1448668).

### Measurement of amino acids:

Amino acids were determined according Fekkes D, van Dalen A, Edelman M, Voskuilen A "Validation of the determination of amino acids in plasma by high performance liquid chromatography using automated pre-column derivatization with o-phtaldialdehyde". Journal of Chrom. B. (1995) 177-186. Cysteine cannot be measured by this method.

### Area under the curve, peak time, maximal peak height:

Area under the curve for glucose and insulin was calculated per animal, during the early peak (t=0-30 min) and under the entire curve measured (t=0-120 min). Negative values (when a respose reaches levels below basal) were subtracted. Per animal the time and level of glucose and insulin peak was determined (maximum plasma concentrations of all measured time points).

### Statistics:

GraphPad InStat software was used to perform statistics. Since data were not normally distributed, differences were tested using the distribution-free Kruskal-Wallis test (paired samples, repeated measures) with post-hoc tests. P-values < 0.05 were considered statistically significant.

**Table 1: Composition of the meals, per 100 ml**

| Composition | Human milk | Nutrilon^{®} 1 |
|---|---|---|
| Protein | 1.1 g | 1.4 g |
| Lactose | 7.0 g | 7.3 g |
| Fat | 4.5 g | 3.5 g |

### Results:

The post-prandial peak of glucose as well as insulin was lower in rats fed human milk, than in rats fed standard infant milk formula (Nutrilon^{®} 1) as can be seen in figure 1A and 1B. The area under the curve (AUC) of insulin and glucose was lower for human milk fed rats than for rats fed with standard infant milk formula as can be seen in table 2. Surprisingly, when a standard infant milk formula supplemented with protease inhibitor was fed, a lower post-prandial peak of both glucose and insulin was observed (Figure 1A and 1B). Also the peak time, maxima peak height, and AUC was lowered (Table 2).

**Table 2: Effects of standard infant formula (Nutrilon^{®} 1), standard infant formula with protease inhibitor and human milk on post-prandial peak time, maximal peak height and area under the curve of glucose and insulin. IMF = infant milk formula, PI = protease inhibitor, HM = human milk.**

| | IMF | IMF+PI | HM |
|---|---|---|---|
| Effect | | | |
| Peak time (m ± se) | | | |
| Glucose | 18.5 ± 8.3 | 9.5 ± 2.7 | 12.0 ± 2.4 |
| | | | |
| Insulin | 10.0 ± 2.5 | 13.3 ± 2.2 | 11.7 ± 1.2 |

| | | | |
|---|---|---|---|
| Maximal peak height (g/l ± se) | | | |
| | | | |
| Glucose | 0.42 ± 0.06 | 0.30 ± 0.04 | 0.33 ± 0.08 |
| | | | |
| Insulin | 1.96 ± 0.32 | 1.59 ± 0.46 | 1.41 ± 0.27 |
| | | | |

| AUC 0-30 (g/l)(% ± se) | | | |
|---|---|---|---|
| | | | |
| Glucose | 6.4 ± 1.7 | 4.8 ± 0.8 | 5.1 ± 1.8 |
| | | | |
| Insulin | 23.4 ± 5.8 | 21.9 ± 5.2 | 19.0 ± 3.2 |
| | | | |

| AUC 0-120 (g/l)(% ± se) | | | |
|---|---|---|---|
| | | | |
| Glucose | 16.8 ± 6.5 | 9.6 ± 3.1 | 11.7 ± 4.6 |
| | | | |
| Insulin | 44.4 ± 14.3 | 35.7 ± 8.0 | 40.1 ± 10.8 |

The amounts of essential amino acids and total amino acids in the blood was determined. The peak of amino acids (at t=5) was highest when IMF is fed. The peak was lowest when human milk is fed. IMF with protease inhibitors showed in general an intermediate effect, see table 3. At t=120 min blood amino acid levels of IMF and human milk fed rats were similar. The levels were a little lower (but not statistically significant) when IMF plus protease inhibitor was fed. Also the AUC values of all amino acids tested were not statistically different between the three groups.

**Table 3: Effects of standard infant formula (Nutrilon^{®} 1), standard infant formula with protease inhibitor and human milk on relative post-prandial peak levels (t=5) of essential and total amino acids except cysteine. The value at t=0 was set at 100 %. IMF = infant milk formula, PI = protease inhibitor, HM = human milk.**

| Amino acid | IMF | IMF+PI | HM |
|---|---|---|---|
| % ± se | | | |
| Val | 114.9 ± 3.5 | 102.8 ± 3.2 | 104.9 ± 4.6 |
| Trp | 127.2 ± 3.0 | 115.9 ± 4.6 | 115.4 ± 7.4 |
| Leu | 117.8 ± 3.2 | 104.1 ± 6.0 | 106.5 ± 6.1 |
| Ile | 116.4 ± 2.9 | 105.5 ± 3.3 | 97.0 ± 1.8 |
| Thr | 115.8 ± 3.1 | 106.2 ± 4.2 | 99.5 ± 3.7 |
| His | 107.4 ± 3.4 | 98.4 ± 2.5 | 97.7 ± 4.5 |
| Tyr | 113.5 ± 5.7 | 106.5 ± 3.7 | 103.0 ± 6.0 |
| Phe | 110.7 ± 2.5 | 102.6 ± 3.0 | 100.7 ± 5.0 |
| Met | 112.2 ± 2.9 | 103.8 ± 2.6 | 102.9 ± 2.6 |
| Total amino acids | 113.3 ± 3.0 | 103.8 ± 2.6 | 100.0 ± 4.7 |

It can be concluded that the presence of a protease inhibitor in infant milk formula resulted in glucose as well as insulin levels and kinetics more similar to those observed with human milk, while the effect on bioavailability of all the amino acids tested was insignificant. These results are indicative for the use of protease inhibitor for the treatment and/or prevention of childhood obesity and secondary disorders caused by childhood obesity.

### Example 2:

A composition (Nutrilon 1^{®}, Nutricia, Zoetermeer, The Netherlands) comprising per 100 ml:

| | | |
|---|---|---|
| Energy: | | 67 kcal |
| Protein: | | 1.4 g cow' milk protein, ratio whey/casein 8/6 |
| Digestible carbohydrates | | 7.5 g, of which 7.3 g lactose |
| Fat | | 3.5 g |
| | Saturated | 1.5 g |
| | Monounsaturated | 1.5 g |
| | Polyunsaturated | 0.5 g, of which 0.4 g LA, and 0.07 g ALA |
| Non digestible, fermentable carbohydrates | | 0.4 g TOS and polyfructose |
| zinc gluconate | | 3.5 mg |
| choline | | 7.6 mg |
| taurine | | 6.3 mg |

### Example 3:

A composition (Nutrilon 2^{®}, Nutricia Zoetermeer, The Netherlands) comprising per 100 ml:

| | | |
|---|---|---|
| Energy: | | 77 kcal |
| Protein: | | 1.49g cow' milk protein, whey/casein 4/15 wt/wt. |
| Digestible carbohydrates | | 9.9 g, of which 6.6 g lactose |
| Fat | | 3.3 g |
| | Saturated | 1.4 g |
| | Monounsaturated | 1.4 g |
| | Polyunsaturated | 0.5 g of which 0.37 g LA and 0.07 g |
| ALA | | |
| Non digestible, fermentable carbohydrates | | 0.4 g TOS and polyfructose |
| choline | | 8.3 mg |
| Egg white trypsin inhibitor (SigmaT2011) | | 5.0 mg |

## Claims

1. A composition comprising 5 to 16 en.% protein, 35 to 60 en.% fat, wherein at least one fat source selected from the group consisting of vegetable fat, marine fat and microbial fat, and 25 to 75 en.% carbohydrates; **characterised in that** the composition comprises one or more protease inhibitors selected from the group consisting of trypsin inhibitor, chymotrypsin inhibitor, and elastase inhibitor.

2. The composition according to claim 1 said composition comprising at least vegetable fat.

3. The composition according to any one of the preceding claims, wherein the protease inhibitor is obtained from a source selected from the group consisting of potato, leguminous seeds, non-human milk, avian egg white and microbial culture

4. The composition according to any of the preceding claims, wherein the protease inhibitor is selected from the group consisting of α₁-antitrypsin, or α₁-proteinase inhibitor, from human plasma; α₁-antichymotrypsin from human plasma; bovine Kunitz trypsin inhibitor from colostrum trypsin-chymotrypsin inhibitor, Bowman-Birk inhibitor, from soy bean; trypsin inhibitor, type II-O, or ovoinhibitor, from chicken egg white; serpin from *Bifidobacteria*, potato inhibitor I; potato inhibitor II; and zinc gluconate.

5. The composition according to claim 1-4, wherein the protease inhibitor is present from 35 µg to 14 mg per g dry weight of the composition.

6. The composition according to claim 1-5, wherein the protease inhibitor is present from 0.75 to 300 protease inhibitor units per g dry weight of the composition, wherein one protease inhibitor unity is the activity that inhibits one unit of the activity of the sum of trypsin activity, chymotrypsin activity and elastase activity.

7. The composition according to claim 1-6, wherein the protease inhibitor is present from 20 to 5000% of the protease inhibitor activity as obtained by 1.1 mg human serum α₁-antitrypsin and 0.18 mg human serum α₁-antichymotrypsin per g dry weight of the composition.

8. The composition according to claim 1-7, wherein at least 50 wt.% of the digestible carbohydrates is lactose.

9. The composition according to claim 1-8 wherein the composition further comprises 1-10 wt.% non-digestible, fermentable carbohydrates selected from the group consisting of trans-galacto-oligosaccharides, inulin, fructo-oligosaccharides, galacturonic acid oligosaccharides, partially hydrolysed guar gum and indigestible polydextrose, based on dry weight of the composition.

10. A process for preparing an infant nutrition, comprising admixing one or more protease inhibitors selected from the group consisting of trypsin inhibitor, chymotrypsin inhibitor, and elastase inhibitor, with protein, carbohydrates and vegetable fat in such amounts that the infant nutrition comprises 5 to 16 en.% protein, 35 to 60 en.% fat and 25 to 75 en.% carbohydrates.

11. Use of at least one protease inhibitor selected from the group consisting of trypsin inhibitor, chymotrypsin inhibitor and elastase inhibitor, for the manufacture of a composition for the treatment and/or prevention of childhood obesity, said composition further comprising 5 to 16 en.% protein, 35 to 60 en.% fat, wherein at least one fat source selected from the group consisting of vegetable fat, marine fat and microbial fat, and 25 to 75 en.% carbohydrates.

12. Use of the composition according to any one of claim 1-9 for the manufacture of a preparation for use in a method to prevent and/or treat childhood obesity, said method comprising orally administering said preparation to an infant.

13. Use of a composition according to any one of claims 1-9 for the manufacture of a composition for providing nutrition to an infant.

14. Use of a composition according to claim 1-9, for the manufacture of a nutritional composition for use in a method for the treatment and/or prevention of diabetes, cardiovascular diseases, hypertension, asthma, sleep apnoea, orthopaedic disorders and/or arthritis, in a subject suffering from childhood obesity, said method comprising orally administering to an infant said nutritional composition.

## Patentansprüche

1. Zusammensetzung umfassend 5 bis 16% En.-% Protein, 35 bis 60% En.-% Fett, worin mindestens eine aus der Gruppe bestehend aus Pflanzenfett, aus dem Meer und aus Mikroorganismen stammendem Fett ausgewählte Fettquelle und 25 bis 75% En.-% Kohlenhydrate,
**dadurch gekennzeichnet, dass**
die Zusammensetzung einen oder mehrere aus der Gruppe bestehend aus Trypsin-Inhibitor, Chymotrypsin-Inhibitor und Elastase-Inhibitor ausgewählte(n) Protease-Inhibitor(en) umfasst.

2. Zusammensetzung nach Anspruch 1, wobei die Zusammensetzung mindestens Pflanzenfett umfasst.

3. Zusammensetzung nach einem der vorangehenden Ansprüche, worin der Protease-Inhibitor aus einer aus der Gruppe bestehend aus Kartoffel, Leguminosensamen, nicht vom Menschen stammender Milch, Geflügeleiweiß und mikrobiellen Kulturen ausgewählten Quelle erhalten wird.

4. Zusammensetzung nach einem der vorangehenden Ansprüche, worin der Protease-Inhibitor aus der Gruppe bestehend aus α₁-Antitrypsin oder α₁-Proteinase-Inhibitor aus menschlichem Plasma, α₁-Antichymotrypsin aus menschlichem Plasma, Trypsin-Inhibitor vom Kunitz-Typ aus dem Trypsin-Chymotrypsin-Inhibitor des Kolostrums der Kuh, Bowman-Birk-Inhibitor aus der Sojabohne, Trypsin-Inhibitor vom Typ II-O oder Ovoinhibitor aus Hühnereiweiß, Serpin aus Bifidobakterien, Kartoffel-Inhibitor-I, Kartoffel-Inhibitor-II und Zinkgluconat ausgewählt ist.

5. Zusammensetzung nach Anspruch 1 bis 4, worin der Protease-Inhibitor in einer Menge zwischen 35 µg und 14 mg pro g Trockengewicht der Zusammensetzung vorliegt.

6. Zusammensetzung nach Anspruch 1 bis 5, worin der Protease-Inhibitor in einer Menge zwischen 0,75 und 300 Protease-Inhibitor-Einheiten pro g Trockengewicht der Zusammensetzung vorliegt, worin eine Protease-Inhibitor-Einheit die Aktivität ist, die eine Aktivitäts-Einheit der Summe der Trypsin-, Chymotrypsin- und Elastase-Aktivitäten hemmt.

7. Zusammensetzung nach Anspruch 1 bis 6, worin der Protease-Inhibitor in einer Menge zwischen 20 bis 5000% der Protease-Inhibitor-Aktivität, wie sie aus 1,1 mg α₁-Antitrypsin aus menschlichem Serum und 0,18 mg α₁-Antichymotrypsin aus menschlichem Serum erhalten wird, pro g Trockengewicht der Zusammensetzung vorliegt.

8. Zusammensetzung nach Anspruch 1 bis 7, worin mindestens 50 Gew.-% der verdaulichen Kohlenhydrate Lactose ist.

9. Zusammensetzung nach Anspruch 1 bis 8, worin die Zusammensetzung ferner 1 bis 10 Gew.-% aus der Gruppe bestehend aus Transgalacto-Oligosacchariden, Inulin, Fructo-Oligosacchariden, Galacturonsäure-Oligosacchariden, teilweise hydrolysiliertem Guargummi und unverdaulicher Polydextrose ausgewählte unverdauliche, gärfähige Kohlenhydrate umfasst, basierend auf dem Trockengewicht der Zusammensetzung.

10. Verfahren zur Herstellung von Säuglingsnahrung umfassend das Zumischen eines oder mehrerer aus der Gruppe bestehend aus Trypsin-Inhibitor, Chymotrysin-Inhibitor und Elastase-Inhibitor ausgewählten Protease-Inhibitors bzw. ausgewählter Protease-Inhibitoren zu Protein, Kohlenhydraten und Pflanzenfett in solchen Mengen dass die Säuglingsnahrung 5 bis 16% En.-% Protein, 35 bis 60% En.-% Fett und 25 bis 75% En.-% Kohlenhydrate umfasst.

11. Verwendung von mindestens einem aus der Gruppe bestehend aus Trypsin-Inhibitor, Chymotrypsin-Inhibitor und Elastase-Inhibitor ausgewählten Protease-Inhibitor zur Herstellung einer Zusammensetzung zur Behandlung und/oder Verhütung der Fettleibigkeit im Kindesalter wobei die Zusammensetzung 5 bis 16% En.-% Protein, 35 bis 60% En.-% Fett, worin mindestens eine aus der Gruppe bestehend aus Pflanzenfett, aus dem Meer und aus Mikroorganismen stammendem Fett ausgewählte Fettquelle und 25 bis 75% En.-% Kohlenhydrate umfasst.

12. Verwendung der Zusammensetzung nach einem der Ansprüche 1 bis 9 zur Herstellung einer Zubereitung zur Verwendung in einem Verfahren zur Verhütung und/oder Behandlung von Fettleibigkeit im Kindesalter, wobei das Verfahren die orale Verabreichung der Zubereitung an einen Säugling umfasst.

13. Verwendung einer Zusammensetzung nach einem der Ansprüche 1 bis 9 zur Herstellung einer Zusammensetzung zur Bereitstellung von Säuglingsnahrung.

14. Verwendung einer Zusammensetzung nach Anspruch 1 bis 9 zur Herstellung einer Nährstoffzusammensetzung zur Verwendung in einem Verfahren zur Behandlung und/oder Verhütung von Diabetes, kardiovaskulären Erkrankungen, Hypertonie, Asthma, Schlafapnoe, orthopädischen Störungen und/oder Arthritis bei einem an Fettleibigkeit im Kindesalter leidenden Patienten, wobei das Verfahren die orale Verabreichung der Nährstoffzusammensetzung an einen Säugling umfasst.

## Revendications

1. Une composition comprenant de 5 à 16 en.% de protéine, de 35 à 60 en.% de matière grasse, dans laquelle au moins une source de matière grasse sélectionnée dans le groupe consistant en les matières grasses végétales, les matières grasses marines, et les matières grasses microbiennes, et de 25 à 75 en.% de glucides, **caractérisé en ce que** la composition comprend un ou plusieurs inhibiteurs de protéase sélectionné parmi le groupe consistant en les inhibiteurs de trypsine, les inhibiteurs de chymothrypsine, et les inhibiteurs d'élastase.

2. La composition selon la revendication 1 laquelle composition comprend au moins de la matière grasse végétale.

3. La composition selon l'une quelconque des revendications précédentes, dans laquelle l'inhibiteur de protéase est obtenu à partir d'une source sélectionnée parmi le groupe consistant en la pomme de terre, les graines légumineuses, le lait non humain, le blanc d'oeuf aviaire, et la culture microbienne.

4. La composition selon l'une quelconque des revendications précédentes, dans laquelle l'inhibiteur de protéase est sélectionné parmi le groupe consistant en l'α₁-antitrypsin, ou l'α₁- inhibiteur de protéinase de plasma humain; l'α₁-antichymotrypsine de plasma humain; inhibiteur de trypsine Kunitz de bovins de l'inhibiteur de trypsin-chymotrypsine colostrum, l'inhibiteur de Bowman-Birk, de soja; l'inhibiteur de trypsine, type II-O, ou ovoinhibiteur, de blanc d'oeufs de poules; la serpine *Bifidobacterie*, l'inhibiteur I de pomme de terre; l'inhibiteur II de pomme de terre; et le gluconate de zinc.

5. La composition selon l'une des revendications 1 à 4, dans laquelle l'inhibiteur de protéase est présent de 35 µg à 14 mg par g de masse sèche de la composition.

6. La composition selon l'une des revendications 1 à 5, dans laquelle l'inhibiteur de protéase est présent de 0.75 à 300 unités d'inhibiteur de protéase par g de masse sèche de la composition, dans laquelle une unité d'inhibiteur de protéase est l'activité qui inhibe une unité de l'activité de la somme des activités de la trypsine, de la chymotrypsine, et de l'élastase.

7. La composition selon l'une des revendications 1 à 6, dans laquelle l'inhibiteur de protéase est présent de 20 à 5000% de l'activité de l'inhibiteur de protéase tel qu'obtenu par 1,1 mg de sérum humain d'α₁-antitrypsine et 0,18 mg de sérum humain d'α₁-antichymotrypsine par g de masse sèche de la composition.

8. La composition selon l'une des revendications 1 à 7, dans laquelle au moins 50% en poids des carbohydrates digestibles est du lactose.

9. La composition selon l'une des revendications 1 à 8, dans laquelle la composition comprend en outre 1 à 10% en poids de glucides fermentables non digestibles sélectionnés parmi le groupe consistant en des trans-galacto-oligosaccharides, de l'inuline, des fructo-oligosaccharides, des oligosaccharides d'acide galacturonique, de la gomme de guar partiellement hydrolysée et du polydextrose indigestible, basé sur la masse sèche de la composition.

10. Un procédé pour la préparation de nutrition d'un nourrisson, comprenant l'adjonction d'un ou plusieurs inhibiteurs de protéase sélectionnés parmi le groupe consistant en un inhibiteur de trypsine, un inhibiteur de chymotrypsine, et un inhibiteur d'élastase, avec des protéines, glucides et matières grasses végétales dans des quantités telles que la nutrition de nourrisson comprend de 5 à 16 en.% de protéines, de 35 à 60 en.% de matières grasses, et de 25 à 75 en.% de glucides.

11. Utilisation d'au moins un inhibiteur de protéase sélectionné parmi le groupe consistant en un inhibiteur de trypsine, un inhibiteur de chymotrypsine, et un inhibiteur d'élastase, pour la fabrication d'une composition pour le traitement et/ou la prévention de l'obésité infantile, ladite composition comprenant en outre de 5 à 16 en.% de protéine, de 35 à 60 en.% de matière grasse, dans laquelle au moins une source de matière grasse est sélectionnée parmi le groupe consistant en des matières grasses végétales, des matières grasses marines, et des matières grasses microbiennes, et de 25 à 75 en.% de glucide.

12. Utilisation d'une composition selon l'une quelconque des revendications 1 à 9 pour la fabrication d'une préparation destinée à être utilisée dans une méthode de prévention et/ou de traitement de l'obésité infantile, ladite méthode comprenant l'administration orale de ladite préparation chez un nourrisson.

13. Utilisation d'une composition selon l'une quelconque des revendications 1 à 9, pour la fabrication d'une composition pour la nutrition d'un nourrisson.

14. Utilisation d'une composition selon l'une des revendications 1 à 9, pour la fabrication d'une composition nutritionnelle destinée à être utilisée dans une méthode de traitement et/ou prévention du diabète, de maladies cardiaques, d'hypertension, d'asthme, d'apnées du sommeil, de dérangements orthopédiques et/ou d'arthrite, chez un sujet souffrant d'obésité infantile, ladite méthode comprenant l'administration orale chez un nourrisson de ladite composition nutritionnelle.
